(19) Europäisches Patentamt
European Patent Office
Office européen des brevets

(11) Veröffentlichungsnummer: **0 543 201 A2**

(12) # EUROPÄISCHE PATENTANMELDUNG

(21) Anmeldenummer: **92118684.7**

(22) Anmeldetag: **31.10.92**

(51) Int. Cl.⁵: **A61K 31/37**, A61K 31/235, A61K 31/245, C07D 407/12, C07C 215/16, C07C 69/78

(30) Priorität: **09.11.91 DE 4136900**

(43) Veröffentlichungstag der Anmeldung: **26.05.93 Patentblatt 93/21**

(84) Benannte Vertragsstaaten: **BE CH DE DK ES FR GB IT LI LU NL**

(71) Anmelder: **SCHAPER & BRÜMMER GMBH & CO. KG**
**Bahnhofstrasse 45**
**W-3320 Salzgitter 61(DE)**

(72) Erfinder: **Beuscher, Norbert, Dr.rer.nat.**
**Katzenwiesenring 22**
**3320 Salzgitter 51(DE)**
Erfinder: **Ritter, Helmut, Prof.rer.nat**
**Bergische Universitat Wuppertal**
**Gaussstrasse 20, 5600 Wuppertal(DE)**
Erfinder: **Bodinet, Cornelia**
**Ernst-Reuter-Strasse 3**
**3320 Salzgitter 51(DE)**

(74) Vertreter: **Lins, Edgar, Dipl.-Phys. et al**
**Patentanwälte Gramm + Lins**
**Theodor-Heuss-Strasse 1**
**W-3300 Braunschweig (DE)**

(54) **Alkoxylierte Phenyl- und Cumarinderivate, deren Herstellung und Verwendung zur Herstellung von Arzneimitteln gegen Virusinfektionen.**

(57) Es wird die Verwendung von alkoxylierten Phenyl- bzw. Cumarinderivaten der Formel

$R^1 - X - R^2$ (I)

in der X eine aliphatische, heteroaliphatische mit 1 bis 2 O-Atomen oder cycloaliphatische Gruppe mit 2 bis 12 C-Atomen ist sowie $R^1$ eine lumarin- und $R^2$ eine substituierte Phenolgruppe ist, zur Herstellung eines Arzneimittels zur Therapie von Virusinfektionen vorgeschlagen.

EP 0 543 201 A2

Gegenstand der Erfindung ist die Verwendung von alkoxylierten Phenyl- bzw. Cumarinderivaten der Formel

$R^1 - X - R^2$ (I)

in der X eine aliphatische, heteroaliphatische mit 1 bis 2 O-Atomen oder cycloaliphatische Gruppe mit 2 bis 12 C-Atomen ist und $R^1$ durch die Formeln

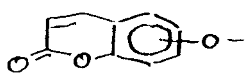

(Ia) oder

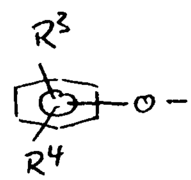

(Ib)

beschrieben wird, wobei $R^3$ für Wasserstoff, eine Hydroxy- oder Alkoxygruppe oder ein Carbonsäurederivat steht, $R^4$ eine Carbonsäure-, Alkylcarbonsäure- oder Acrylsäurefunktion bzw. eines ihrer Derivate darstellt, Und $R^2$ ein Rest aus der Gruppe $R^1$, eine Hydroxygruppe, Bromid, Chlorid, Alkoxycarbonyl mit 1 bis 6 C-Atomen, eine unsubstituierte oder substituierte Aminofunktion oder ein Oxyphenylrest der Formel

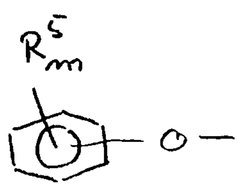

(Ic)

ist, wobei m eine ganze Zahl zwischen 1 und 5 und $R^5$ ein Alkyl- oder Oxyalkylrest darstellt, zur Herstellung eines Arzneimittels zur Therapie von Virusinfektionen, insbesondere Influenza und Rhinitis acuta.

Weiterhin ist ein Arzneimittel, alkoxylierte Phenyl- bzw. Cumarinderivate der Formel

$R^6 - X - R^7$ (II)

enthaltend, in der X eine aliphatische, heteroaliphatische mit 1 bis 2 O-Atomen oder cycloaliphatische Gruppe mit 2 bis 12 C-Atomen ist und $R^6$ durch die Formeln

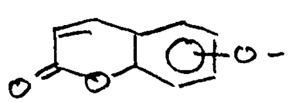

(IIa) oder

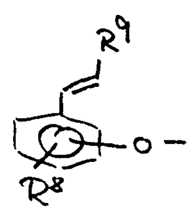

(IIb)

beschrieben wird, wobei $R^8$ für Wasserstoff, eine Hydroxy- oder Alkoxygruppe oder ein Carbonsäurederivat steht, $R^9$ eine Carbonsäure- oder Alkylcarbonsäurefunktion bzw. eines ihrer Derivate darstellt, und $R^7$ ein Rest aus der Gruppe $R^6$, eine Hydroxygruppe, Bromid, Chlorid, Alkoxycarbonyl mit 1 bis 6 C-Atomen, eine unsubstituierte oder substituierte Aminofunktion oder ein Oxyphenylrest der Formel

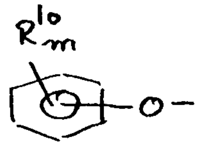

(IIc)

ist, wobei m eine ganze Zahl zwischen 1 und 5 und $R^{10}$ einen Alkyl – oder Oxyalkylrest darstellt, Gegenstand der Erfindung.

Picornaviren rufen beim Menschen eine Reihe von Krankheitsbildern unterschiedlichen Schweregrades hervor. Darunter fallen lebensbedrohliche Infektionen wie Poliomyelitis, Hepatitis A, Influenza und leichtere, wie der Schnupfen. Dieser wird häufig durch Rhinoviren hervorgerufen, von denen zur Zeit über 100 Serotypen bekannt sind.

Es sind bis heute zahlreiche Substanzen entwickelt worden, die gegen Rhinoviren eine gewisse Aktivität aufweisen. Die Strukturen und Eigenschaften solcher Verbindungen sind in verschiedenen Arbeiten, z. B. in M. S. Chapman et al., J. Mol. Biol. (1991), 217, S. 455 – 463, vorgestellt worden. Obwohl einige der Verbindungen, z. B. ein Oxazolinyl – Isoxazol oder ein phosphoryliertes Chalkon – Derivat oder 4,6' – Dichlorflavan eine hervorragende Wirksamkeit in vitro aufweisen, ist jedoch eine therapeutische Anwendung entweder wegen fehlender Aktivität in – vivo oder wegen erheblicher Nebenwirkungen z. B. starker Reizung der Schleimhäute in der Nase, leider bisher nicht gelungen (vgl. P. G. Higgens et al., The British Soc. for Antimicrobial Chemotherapy, (1984), S. 403 – 409).

Bemerkenswert ist auch eine Beobachtung von G. D. Diana et al. (J. Med. Chem. (1985), 28, S. 1906 – 1910), daß Isoxazolabkömmlinge eine gewisse antivirale Wirksamkeit, z. B. gegen Rhinoviren, aufweisen. Eine Anwendung dieser Isoxazole enthaltenden Substanzgruppen ist bisher leider ebenfalls nicht erfolgt.

Die vorliegende Erfindung behinhaltet die Verwendung von alkoxylierten Phenyl – und Cumarinderivaten der Formel (I) für die Herstellung von Arzneimitteln zur therapeutischen Behandlung von Infektionen, hervorgerufen durch Picornaviren, insbesondere zur Bekämpfung von Viruserkrankungen, welche durch Rhinoviren ausgelöst wurden.

Die Substanzen gemäß Formel (I) können in üblicher Weise galenisch formuliert werden und oral, i.v., i.m. oder topisch appliziert werden. Hierbei kommen die üblichen Applikationsformen in Fragen, wie beispielsweise Tabletten, Kapseln, Dragees, Sirupe, Lösungen, Suspensionen, Sprays oder Salben. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z. B. Tartrat – und Citratpuffer, Ethanol, Komplexbildner wie Ethylendiaminotetraessigsäure und deren nichttoxische Salze sowie hochmolekulare Polymere wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Manit, Methylcellulose, Talkum, hochdisperse Kieselsäure, höhermolekulare Fettsäuren wie Stearinsäure, Gelatine, Agar – Agar, Calciumphosphat, Magnesiumstearat, tierische und pflanzliche Fette, feste hochmolekulare Polymere wie Polyethylenglykole und andere chemische Verbindungen.

Nach bekannten Verfahren lassen sich von den beanspruchten Verbindungen ebenfalls Cyclodextrin – Einschlüsse herstellen, die zu einer verbesserten Löslichkeit der Verbindungen in wäßrigem Milieu führen. Neben den unmodifizierten Cyclodextrinen eignen sich besonders auch die alkylierten Cyclodextrin – Derivate, wie z. B. Dimethyl – $\beta$ – cyclodextrin.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 bis 100 mg, vorzugsweise 0,2 bis 80 mg pro Tag und pro Kilogramm Körpergewicht appliziert. Bevorzugt ist es, die Tagesdosis auf zwei bis fünf Applikationen zu verteilen, wobei bei jeder Applikation ein bis zwei Tabletten mit einem Wirkstoffgehalt von 0,5 bis 500 mg verabreicht werden. Die Tabletten können auch retardiert sein, wodurch sich die Anzahl der Applikationen pro Tag auf ein bis drei vermindert. Der Wirkstoffgehalt der retardierten Tabletten kann 2 bis 1000 mg betragen. Der Wirkstoff kann auch durch Dauerinfusion gegeben werden, wobei die Mengen von 5 bis 1000 mg pro Tag normalerweise ausreichen. Bei topischer Applikation kann bis zu fünfmal täglich appliziert werden, vorzugsweise in einer Menge von 0,1 bis 50 mg.

Die Synthesen der beanspruchten Verbindungen, die zum großen Teil bisher noch nicht bekannt waren, erfolgen nach dem Fachmann bekannten Verfahren. Folgende Beispiele sind charakteristisch für die Herstellung der Verbindungen:

Synthesebeispiel 1: Hexamethylen – bis(7 – oxycumarin)

8,92 g (55 mmol) Umbelliferon (7 – Hydroxy – cumarin) und 7,60 g (55 mmol) $K_2CO_3$ werden in 250 ml Aceton suspendiert und anschließend 6,10 g (25 mmol) Hexamethylendibromid zugesetzt. Die Reaktionsmischung wird 30 Stunden unter Rückfluß erhitzt. Dabei wird die Suspension zunehmend dünnflüssiger. Nach Abkühlen auf Raumtemperatur wird die Reaktionsmischung in 300 ml Wasser eingegossen und mit verdünnter Salzsäure sauer gestellt. Der feste Rückstand wird abgesaugt und mit Wasser gewaschen. Das

Rohprodukt wird mit 100 ml Ethanol verrührt und die Kristalle werden erneut abgesaugt. Das farblose Produkt wird schließlich im Vakuum über $P_4O_{10}$ getrocknet.
Ausbeute: 5,6 g (55 %); Schmelzpunkt: 162 − 163 °C
IR (KBr): 3050, 3045 (CH, aromatisch); 2940, 2905, 2870 (CH, aliphatisch); 1715 (C=O) 1620 (C=C) 1605 (Aromat C=C), jeweils in $cm^{-1}$.
H-NMR ($CDCl_3$, 250 MHz); 1,55 m (4H); 1,84 m (4H), 4,01 t (4H), 6,21 d (2H), 6,75 − 1,83 m (4H), 7,34 d (2H), 7,61 d (2H), jeweils in ppm.

Synthesebeispiel 2: 6-Bromhexoxy-(4-benzoesäureethylester)

16,6 g (100 mmol) 4-Hydroxybenzoesäureethylester, 122 g (500 mmol) 1,6-Dibromhexan, 10,0 g $K_2CO_3$ (fein gemörsert und getrocknet) werden in 75 ml absolutiertem Aceton unter Rückfluß erhitzt. Nach 20 Stunden wird zunächst das Aceton und anschließend der Überschuß an Dibromhexan bei 15 Torr abedestilliert. Zur Reinigung wird das Rohprodukt im Hochvakuum mittels einer Kugelrohrdestille gereinigt. Nach Umkristallisieren aus wenig Ethanol und Trocknen im Vakuum über $P_4O_{10}$ erhält man ca. 10 g analysenreines Produkt. Der Schmelzpunkt liegt im Bereich der Raumtemperatur.

Synthesebeispiel 3: 6-(N-Diethanolamino)-hexoxy-(4-benzoesäureethylester)

3,29 g (1 mmol) 6-Bromhexoxy-(4-benzoesäureethylester) (vgl. Synthesebeispiel 2) und 2,2 g (2,1 mmol) Diethanolamin werden in 10 ml Nitromethan 4 Stunden unter Rückfluß erhitzt, danach wird im Vakuum das Lösungsmittel abgedampft. Der Rückstand wird in 40 ml Dichlormethan aufgenommen, mit Wasser gründlich ausgeschüttelt, die organische Phase mit $Na_2SO_4$ getrocknet, filtriert und zur Trockene eingedampft. Der Rückstand wird mit wenig Ether und Petrolether überschichtet. Bei ca. 0 °C kristallisiert das Endprodukt in reiner Form aus. Schmelzpunkt 18 − 20 °C.

Als besonders bevorzugte Strukturen haben sich im Rahmen der vorliegenden Erfindung die folgenden Verbindungen erwiesen:

Hexamethylen-bis(4-oxyzimtsäureethylester)
Hexamethylen-bis(4-oxyzimtsäuremethylester)
Hexamethylen-bis(7-oxycumarin)
Pentamethylen-bis(4-oxyzimtsäureethylester)
1(4-Toluoyloxy)-6-(4-oxybenzoesäureethylester)-hexan
1-(Umbelliferyl)-6-(4-oxybenzoesäureethylester)-hexan
6-Bromhexoxy-(4-benzoesäureethylester)
6-(N-Diethanolamino)-hexoxy-(4-benzoesäureethylester)

Die antivirale Wirkung der erfindungsgemäßen Verbindungen wird durch nachstehende Beispiele erläutert, ohne die Erfindung zu beschränken:

Beispiel 1

Rhinoviren können aufgrund des Rezeptors, an welchen sie binden, in zwei Gruppen aufgeteilt werden. In eine große Gruppe, hierzu gehört das Rhinovirus Typ 2, und in eine kleine Gruppe, zu welcher Rhinovirus Typ 1A gehört. Gegen Rhinovirus Typ 2 zeigten die erfindungsgemäßen Verbindungen folgende Aktivität:

Tabelle 1

| | Konzentration [µg/ml] | Plaquezahl |
|---|---|---|
| Kontrolle | − | 176 |
| Hexamethylen-bis(4-oxybenzoesäureethylester | 25 | 0 |
| Octamethylen-bis(4-oxybenzoesäureethylester | 25 | 21 |
| Hexamethylen-bis(4-oxyzimtsäureethylenester) | 25 | 0 |
| Hexamethylen-bis(4-oxyzimtsäureethylenester) | 12.5 | 0 |
| Hexamethylen-bis(4-oxyzimtsäureethylenester) | 6.25 | 0 |

Gegen Rhinovirus Typ 1A zeigten die erfindungsgemäßen Verbindungen folgende Aktivität:

Tabelle 2

| | Konzentration [µg/ml] | Plaquezahl |
|---|---|---|
| Kontrolle ohne Substanz | – | 168 |
| Hexamethylen-bis(4-oxyzimtsäureethylester) | 12.5 | 0 |
| Hexamethylen-bis(4-oxyzimtsäureethylester) | 6.25 | 47 |

Die Verträglichkeit der erfindungsgemäßen Verbindungen wurde auf verschiedenen Säugetier-Zellinien getestet. Dabei wurde auf Hela-Zellen folgende Ergebnisse erzielt:

Tabelle 3

| | nichttoxische Grenz-Konzentration [µg/ml] |
|---|---|
| Kontrolle ohne Substanz | – |
| Trimethylen-bis(4-oxybenzoesäureethylester) | 200 |
| Tetramethylen-bis(4-oxybenzoesäureethylester) | 200 |
| Hexamethylen-bis(4-oxybenzoesäureethylester) | 50 |
| Hexamethylen-bis(4-oxyzimtsäureethylester) | 200 |
| Octamethylen-bis(4-oxybenzoesäureethylester) | 25 |
| 1(4-Toluoyloxy)-6-(4-oxybenzoesäureethylester) | 200 |
| 1(Umbelliferyl)-6-(4-oxybenzoesäureethylester)-hexan | 50 |
| 6-Bromhexoxy-(4-benzoesäureethylester) | 100 |
| 1-(N-Morpholyl-6-(4-oxybenzoesäureethylester)-hexan | 25 |
| Hexamethylen-bis(7-oxycumarin) | 200 |
| 1-Diethanolamino-6-(4-oxybenzoesäureethylester)-hexan | 12 |

Die bevorzugten Substanzen besitzen somit in der Zellkultur eine sehr gute Verträglichkeit. Diese liegt zum Teil erheblich über den zu 4'-Ethoxy-2'hydroxy-4,6'-dimethylchalcon publizierten Daten (vgl. H. Ishitsuka et al., Antimicrob. Agents and Chemotherapy (1982) 22 (4), 617-621).

Die überraschende antivirale Aktivität der erfindungsgemäßen Wirkstoffe gegenüber Rhinovirus Typ 2 ist weiterhin aus der Tabelle 4 ersichtlich. Der Selektivitätsindex ist dabei auf eine 50% bzw. 100% Plaquereduktion bezogen und ergibt sich für SI 50% aus dem Quotienten der höchsten nicht zytotoxischen Konzentration, die eine mindestens 50%-ige Plaquereduktion bewirkt, und der niedrigsten nicht zytotoxischen Konzentration, die eine mindestens 50%-ige Plaquereduktion bewirkt. Entsprechendes gilt für SI 100% (Mohamed Abou-Karam, W. Thomas Shier, Journal of Natural Products; Vol. 53, No. 2, pp. 340-344, März - April 1990). Die Abkürzung "n.b." in Tabelle 4 hat die Bedeutung: nicht bestimmbar.

Tabelle 4

Antivirale Aktivität gegenüber Rhinovirus Typ 2

| Testsubstanz | "SI 50 %" | "SI 100 %" | wirksamer Konzentrationsber. µg/ml |
|---|---|---|---|
| Umbelliferyl-hexoxy-(2-methylbenzol) | 4 | n.b. | 10 - 2.5 |
| Umbelliferyl-hexoxy-(4-benzonitril) | 4 | n.b. | 10 - 5 |
| Umbelliferyl-hexoxy-(2-nitrobenzol) | 2 | 2 | 25 - 12.5 |
| Umbelliferyl-hexoxy-(4-propiophenon) | 8 | 2 | 50 - 6.25 |
| Umbelliferyl-hexoxy-(3,4-dimethylbenzol) | 8 | 4 | 10 - 1.3 |
| Umbelliferyl-hexoxy-(4-benzophenon) | 2 | 1 | 1.25 - 0.5 |
| Umbelliferyl-hexylbromid | 4 | 2 | 5 - 1.3 |
| Umbelliferyl-hexoxy-(4-nitrobenzol) | 2 | 1 | 2.5 - 1.3 |
| Umbelliferyl-hexoxy-(4-methylbenzol) | 2 | 1 | 2.5 - 1.3 |
| Umbelliferyl-hexoxy-(2-brombenzol) | 2 | 1 | 2.5 - 1.3 |
| Umbelliferyl-hexoxy-benzol | 2 | n.b. | 2.5 - 1.3 |
| Umbelliferyl-hexoxy-4-fluorbenzol | n.b. | n.b. | --- |
| Umbelliferyl-hexoxy-(3-chlor-2-toluol) | 2 | 1 | 50 - 25 |
| Umbelliferyl-hexoxy-4-chlorbenzol | 2 | n.b. | 12.5 -6.25 |

| | | | |
|---|---|---|---|
| Umbelliferyl-hexoxy-3-anisol | 4 | 1 | 50 - 12.5 |
| Umbelliferyl-hexoxy-2-naphthalin | 8 | n.b. | 10 - 1.25 |
| Umbelliferyl-hexoxy-1-naphthalin | 2 | n.b. | 10 - 5 |
| Umbelliferyl-hexoxy-3-methylbenzol | n.b. | n.b. | --- |
| Umbelliferyl-hexoxy-2,6-dimethylbenzol | 2 | 1 | 5 - 2.5 |
| Umbelliferyl-hexoxy-eugenol | 20 | 10 | 2.5-0.125 |
| m-Phenylen-bis (oxyhexyl-umbelliferon) | 2 | n.b. | 5 - 2.5 |
| Umbelliferyl-hexoxy-4-Zimtsäure | 4 | n.b. | 10 - 2.5 |

## Patentansprüche

**1.** Verwendung von alkoxylierten Phenyl – bzw. Cumarinderivaten der Formel

$R^1 - X - R^2$ (I)

in der X eine aliphatische, heteroaliphatische mit 1 bis 2 O – Atomen oder cycloaliphatische Gruppe mit 2 bis 12 C – Atomen ist und $R^1$ durch die Formeln

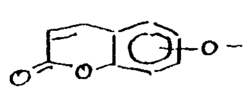

(Ia) oder

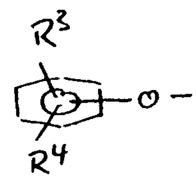

(Ib)

beschrieben wird, wobei $R^3$ für Wasserstoff, eine Hydroxy – oder Alkoxygruppe oder ein Carbonsäurederivat steht, $R^4$ eine Carbonsäure –, Alkylcarbonsäure – oder Acrylsäurefunktion bzw. eines ihrer Derivate darstellt, Und $R^2$ ein Rest aus der Gruppe $R^1$, eine Hydroxygruppe, Bromid, Chlorid, Alkoxycarbonyl mit 1 bis 6 C – Atomen, eine unsubstituierte oder substituierte Aminofunktion oder ein Oxyphenylrest der Formel

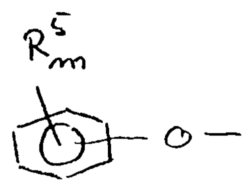

(Ic)

ist, wobei m eine ganze Zahl zwischen 1 und 5 und $R^5$ ein Alkyl – oder Oxyalkylrest darstellt, zur Herstellung eines Arzneimittels zur Therapie von Virusinfektionen, insbesondere Influenza und Rhinitis acuta.

**2.** Verwendung eines Arzneimittels nach Anspruch 1, bei dem $R^4$ eine Carbonsäureamid – , Alkylcarbonsäureamid – oder Acrylsäureamidfunktion ist.

**3.** Verwendung eines Arzneimittels nach Anspruch 1, bei dem $R^4$ eine Carbonsäurealkylester – , Alkylcarbonsäurealkylester – oder Acrylsäurealkylesterfunktion mit 1 bis 10 C – Atomen ist.

**4.** Verwendung eines Arzneimittels nach Anspruch 1, bei dem $R^4$ eine Carbonsäurecyclohexylester – , Alkylcarbonsäurecyclohexylester – oder eine Acrylsäurecyclohexylesterfunktion ist.

**5.** Verwendung eines Arzneimittels nach Anspruch 2, bei dem die Säureamidfunktion einen aliphatischen Substituenten aufweist.

**6.** Verwendung eines Arzneimittels nach Anspruch 2 bei dem die Säureamidfunktion als Substituenten einen Cyclohexylrest aufweist.

**7.** Verwendung eines Arzneimittels nach einem der Ansprüche 1 bis 6, bei dem $R^2$ eine N – Piperidyl – , N – Morpholyl – , N – Diethanolamino – , N – Alkyl – N – ethanolamin – oder N – Dialkylgruppe ist.

**8.** Arzneimittel, alkoxylierte Phenyl – bzw. Cumarinderivate der Formel

$R^6 - X - R^7$ (II)

enthaltend, in der X eine aliphatische, heteroaliphatische mit 1 bis 2 O – Atomen oder cycloaliphatische Gruppe mit 2 bis 12 C – Atomen ist und $R^6$ durch die Formeln

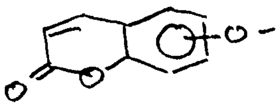

(IIa) oder

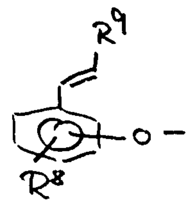

(IIb)

beschrieben wird, wobei $R^8$ für Wasserstoff, eine Hydroxy – oder Alkoxygruppe oder ein Carbonsäurederivat steht, $R^9$ eine Carbonsäure – oder Alkylcarbonsäurefunktion bzw. eines ihrer Derivate darstellt, und $R^7$ ein Rest aus der Gruppe $R^6$, eine Hydroxygruppe, Bromid, Chlorid, Alkoxycarbonyl mit 1 bis 6 C – Atomen, eine unsubstituierte oder substituierte Aminofunktion oder ein Oxyphenylrest der Formel

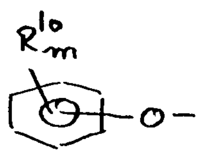

(IIc)

ist, wobei m eine ganze Zahl zwischen 1 und 5 und $R^{10}$ einen Alkyl – oder Oxyalkylrest darstellt.

**9.** Arzneimittel nach Anspruch 8, bei dem $R^4$ eine Carbonsäureamid – , Alkylcarbonsäureamid – oder Acrylsäureamidfunktion ist.

**10.** Arzneimittel nach Anspruch 8, bei dem $R^4$ eine Carbonsäurealkylester – , Alkylcarbonsäurealkylester – oder Acrylsäurealkylesterfunktion mit 1 bis 10 C – Atomen ist.

**11.** Arzneimittel nach Anspruch 8, bei dem $R^4$ eine Carbonsäurecyclohexylester – , Alkylcarbonsäurecyclohexylester – oder eine Acrylsäurecyclohexylesterfunktion ist.

**12.** Arzneimittel nach Anspruch 9, bei dem die Säureamidfunktion einen aliphatischen Substituenten aufweist.

**13.** Arzneimittel nach Anspruch 9, bei dem die Säureamidfunktion als Substituenten einen Cyclohexylrest aufweist.

**14.** Arzneimittel nach einem der Ansprüche 8 bis 13, bei dem $R^2$ eine N – Piperidyl – , N – Morpholyl – , N – Diethanolamino – , N – Alkyl – N – ethanolamin – oder N – Dialkylgruppe ist.